(19) Europäisches Patentamt
European Patent Office
Office européen des brevets

(11) Publication number: 0 159 143
B1

(12) **EUROPEAN PATENT SPECIFICATION**

(45) Date of publication of patent specification: **16.12.87**

(51) Int. Cl.$^4$: **C 07 D 235/02,**
C 07 D 263/52, C 07 D 277/60

(21) Application number: **85301809.1**

(22) Date of filing: **15.03.85**

# (54) Spiro-indenes and spiro-1,2-dihydro-naphthalenes for treatment of diabetic complications.

(30) Priority: **23.03.84 US 593012**

(43) Date of publication of application:
**23.10.85 Bulletin 85/43**

(45) Publication of the grant of the patent:
**16.12.87 Bulletin 87/51**

(84) Designated Contracting States:
**BE DE FR GB IT LU NL**

(56) References cited:
**EP-A-0 079 675**
**EP-A-0 115 133**
**GB-A-2 119 796**
**US-A-4 117 230**
**US-A-4 130 714**
**US-A-4 181 729**
**US-A-4 226 875**

(73) Proprietor: **PFIZER INC.**
**235 East 42nd Street**
**New York, N.Y. 10017 (US)**

(72) Inventor: **Lipinski, Christopher Andrew**
**10 Connshire Drive**
**Waterford, CT (US)**

(74) Representative: **Moore, James William, Dr.**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 159 143 B1**

**Description**

This invention relates to novel spiro-3-heteroazolidinediones useful in the treatment of certain chronic complications arising from diabetes mellitus, such as diabetic cataracts, retinopathy and neuropathy, to pharmaceutical compositions containing such compounds.

In the past various attempts have been made to obtain more effective oral anti-diabetic agents. Generally these efforts have involved synthesis of new organic compounds, particularly sulfonyl ureas, and determination of their ability to substantially lower blood sugar levels when administered orally. However, little is known about the effect of organic compounds in preventing or alleviating chronic complications of diabetes, such as diabetic cataracts, neuropathy and retinopathy. U.S. Patent No. 3,821,383 discloses aldose reductase inhibitors like 1,3-dioxo-1H-benz[d,e]isoquinoline-2(3H)-acetic acid and derivatives thereof to be useful for the treatment of these conditions. U.S. Patent 4,226,875 teaches the use of spiro-oxazolidinediones for treating complications of diabetes as aldose reductase inhibitors. Such aldose reductase inhibitors function by inhibiting the activity of the enzyme aldose reductase, which is primarily responsible for regulating the reduction of aldoses, such as glucose and galactose, to the corresponding polyols, such as sorbitol and galactitol, in humans and other animals. In this way unwanted accumulations of galactitol in the lens of galactosemic subjects and of sorbitol in the lens, peripheral nervous cord and kidneys of various diabetic subjects are prevented or reduced. Accordingly, such compounds are of therapeutic value as aldose reductase inhibitors for controlling certain chronic diabetic complications, including those of an ocular nature, since it is known in the art that the presence of polyols in the lens of the eye leads to cataract formation, with a concomitant loss of lens clarity.

The compounds of the present invention are spiro-3-hetero-azolidindiones of the formula

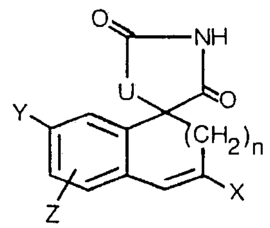

I

and the pharmaceutically acceptable salts thereof, wherein U is oxygen, sulfur or nitrogen substituted with hydrogen; n is zero or one; X is hydrogen or alkyl having 1—4 carbon atoms; Y is hydrogen, halo, nitro, trifluoromethyl, hydroxy, alkoxy or alkylthio having 1—4 carbon atoms or alkyl 1 having 1—4 carbon atoms; Z is hydrogen, halo, nitro, trifluoromethyl, hydroxy, alkoxy having 1—4 carbon atoms or alkyl having 1—4 carbon atoms, with the proviso that if either Y or Z is nitro the other is hydrogen.

Preferred compounds of the invention are those wherein U is nitrogen substituted with hydrogen preferably wherein X, Y and Z are each hydrogen.

Also embraced by the present invention are compounds wherein U is nitrogen substituted by hydrogen, n is one, X is alkyl having 1 to 4 carbon atoms preferably methyl, preferably when Z is hydrogen, more preferably when Y is fluoro.

A group of compounds included in the present invention are those wherein U is oxygen, X is methyl, Y is fluoro and Z is hydrogen.

Another included group of compounds within the present invention are those wherein U is sulfur, X is methyl, Y is fluoro and Z is hydrogen.

Both mixtures of optically active isomers and partially or completely optically resolved isomers of the compounds claimed herein are within the scope of the present invention.

Also comprised by the present invention are pharmaceutical compositions comprising a pharmaceutically acceptable carrier and a compound of formula I. Preferred compositions are those wherein U is nitrogen substituted with a hydrogen, n is one, X and Z are hydrogen, Y is fluoro and the compound is the 4 I'S enantiomer. Also preferred compositions are those wherein U is nitrogen substituted with a hydrogen, n is one, X is methyl and Y is fluoro, and more preferred, the compound is the 4 1'S negatively rotating enantiomer.

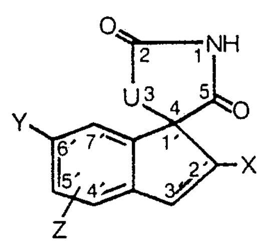

1A

The numbering system of the spiro compounds of formula I where n is zero (IA) is as shown. These

compounds (IA) are 6'-Y-4' or 5'-Z-2'-X-spiro[imidazolidine-, oxazolidine- or thiazolidine-4,1'-inden]2,5-dione derivatives.

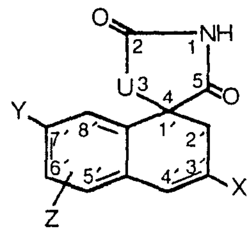

IB

The numbering system of compounds of formula I wherein n is one (IB) is as shown. These compounds are, 7'-Y-6' or 5'-Z-3'-X-spiro[imidazolidine-, oxazolidine- or thiazolidine-4,1'-1',2'-dihydronaphthalene] 2,5-diones.

The compounds of formula II in reaction Scheme A, hereinafter, wherein U is nitrogen substituted with a hydrogen (NH), oxygen (O) or sulfur (S) may be prepared from corresponding 1-indanones and 1-tetralones by well-known methods such as described in U.S. Patent 4,117,230 (U=NH).

Compounds of formula I wherein U is oxygen or sulfur can be prepared in a manner similar to that when U is NH. Of course, the reagents employed with the carbonyl precursor to form the oxazolidinedione ring (U is oxygen) and the thiazolidinedione ring (U=S) are different than the reagents which react to form the hydantoin ring (U is NH). The compounds of formula II wherein U is oxygen are known or can be prepared by the methods described, for example, in U.S. Patent 4,226,875. The synthesis of spiro oxazolidinediones is disclosed in R.C. Schnur et al., *Journal of Medicinal Chemistry,* 25, 1451—4 (1982).

The compounds of formula II are reacted at about 0 to 60°C, preferably about 25°C, with an agent protecting the imide nitrogen such as a perfluorocarboxylimine having N-dialkylsilyl and an O-dialkylsilyl groups, said alkyl containing from 1 to 6 carbon atoms, for example N-dimethylsilyl-O-dimethylsilyl-perfluoro-acetic acid imine, in a nonreative halogenated solvent such as chloroform which does not interfere with the subsequent halogenation reaction.

The *in situ* halogenation is preferably carried out with a molecular halogen such as bromine, chlorine or iodine, more preferably bromine. For example, *in situ* bromination with bromine at a temperature range of about 25 to 100°C, preferably about 60°C results in the 4'-bromo derivative.

The 4'-halo derivative is heated at a temperature range of about 70° to 130°, preferably about 100°C. Before heating, said nonreactive halogenated solvent may be removed. In that case, an inert solvent may be added before heating such as toluene, benzene, higher boiling hydrocarbons such as isooctane or chlorohydrocarbons.

Alternatively, the reaction according to Scheme B below may be employed. The reaction proceeds by heating a compound of formula III with an acid in a reaction-inert solvent at temperatures of from 80° to 150°C. The acid is in general a strong mineral or organic acid such as p-toluene sulfonic acid, hydrochloric acid, hydrobromic acid, hydrofluoric acid, methane sulfonic acid. The reaction-inert solvent is preferably one which refluxes at 80° to 150°C such as toluene, benzene, or higher boiling alcohols such as *n*-butanol, 3-methyl-2-butanol, 3-methyl-n-butanol or isopropyl alcohol.

The corresponding compounds of formula III can be prepared by reacting a corresponding compound of formula II as in reaction scheme A but omitting therefrom the *in situ* heating step (2). The formed compound (having formula II') has a halogen on the 3'-position when n=0 (indene) and the 4'-position when n=1 (1',2'-dihydronaphthalene). This compound is hydrolysed in water having a pH of about 1 to 7 at a temperature of about 0 to 60°C.

Alternatively, the compounds of formula III, wherein U is NH, may be prepared by reaction Scheme C, hereinafter. The corresponding benzene-gamma-oxo acids IV, which are known or can be prepared by procedures analogous to those described for the known compound, are reacted with an ammonium salt such as ammonium carbonate and an alkali metal cyanide such as sodium or potassium cyanide, preferably sodium cyanide, in an aqueous solution such as water or a water-alcohol mixture, e.g., water-ethanol, at a pH of about 9 to 10, preferably 10 and a temperature range of about 50 to 100°C, preferably about 70°C to obtain the compound of formula V (U is NH).

The resulting condensation product V is reacted with a strong Lewis acid such as polyphosphoric acid at a temperature range of 100 to 200°C, preferably about 150°C to obtain VI. Alternatively, concentrated sulfuric acid (6 to 9 M, preferably 9 M) at about 60 to 180°C, preferably about 120°C can be employed. Other strong Lewis acids such as mineral acids, aluminum trichloride and ferric chloride can also be employed.

Scheme A

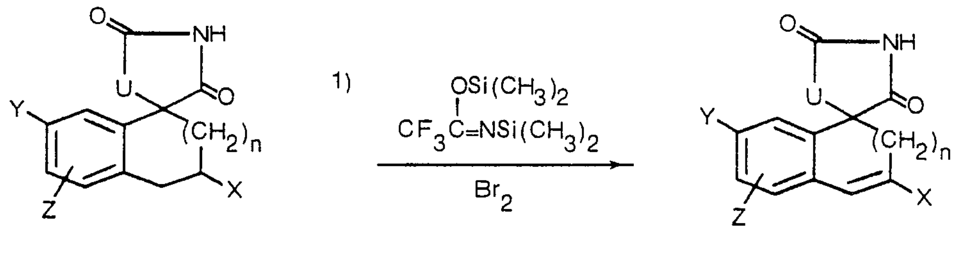


II

I

Scheme B

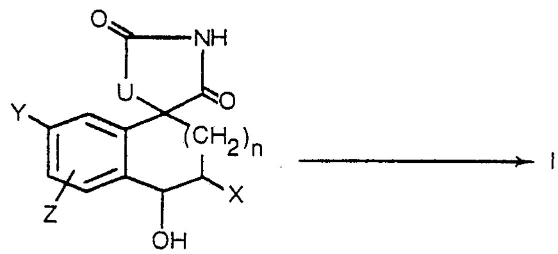

III

Scheme C

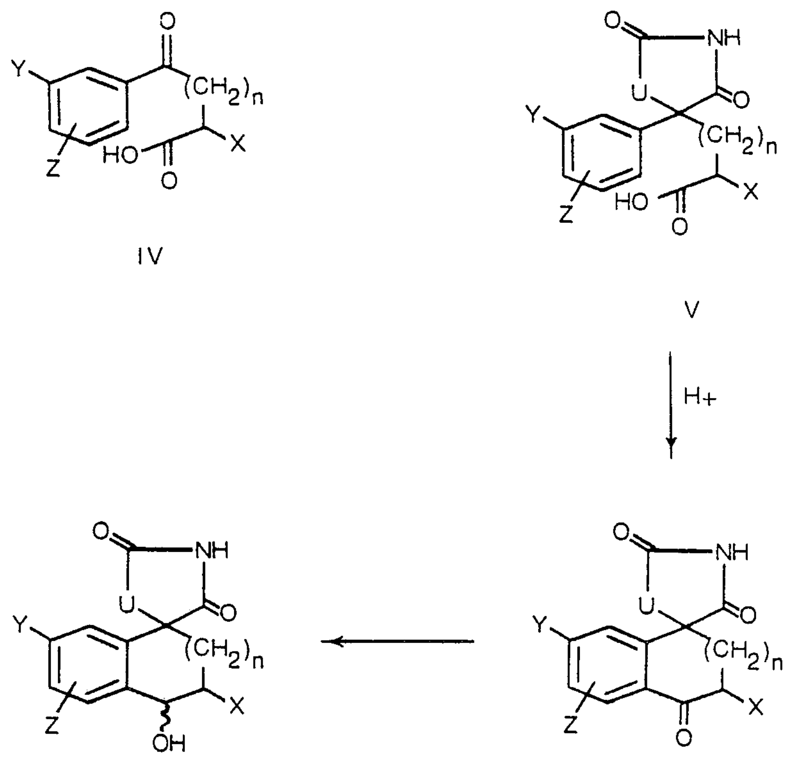


IV

V

VI

III

Compounds of formula III wherein U is oxygen can also be prepared using Scheme C. The corresponding benzene-gamma-oxo acids IV which are known or can be prepared by procedures closely analogous to those described for known compounds are reacted with a trialkysilyl cyanide $(R')_3SiCN$, wherein R' is lower alkyl of 1 to 6 carbon atoms, to form a cyano trialkylsilyloxy derivative according to the general procedure described in United States Patent 4,267,342.

Compounds of formula III wherein U is S can be prepared by Scheme C in a manner similar to that used to prepare the compounds of formula III wherein U is NH. Advantage is taken of the preparation of cyanotrialkylsilyloxy derivatives which are intermediates in the preparation of compounds of formula III wherein U is O. These cyanotrialkylsilyloxy derivatives may be converted by similar methods to thiazolidinedione intermediates of structure II wherein U is S (Scheme A) and intermediates of structure V wherein U is S (Scheme C).

Intermediates of structure II wherein U is S (Scheme A) and intermediates of structure V wherein U is S (Scheme C) may be converted to final products of structure III as previously described for U being NH.

Because of the acidic hydrogen atom in the spiroheterocyclic ring of the compounds of formula I, salts may be formed with pharmaceutically acceptable cations by conventional methods. Thus, these salts may be readily prepared by treating the compound of formula I with an aqueous solution of the desired pharmaceutically acceptable cation and evaporating the resulting solution to dryness, preferably under reduced pressure. Alternatively, a lower alkyl alcohol solution of the compound of formula I may be mixed with an alkoxide of the desired metal and the solution subsequently evaporated to dryness. Suitable pharmaceutically acceptable cations for this purpose include, but are not limited to, alkali earth metal cations such as potassium and sodium, ammonium or water-soluble amine addition salts such as the lower alkanol-ammonium and other base salts with organic amines which are pharmaceutically acceptable and alkaline earth metal cations such as calcium and magnesium.

The novel compounds of formula I and the pharmaceutically acceptable salts thereof are useful as inhibitors of the enzyme aldose reductase in the treatment of chronic complications of diabetes, such as diabetic cataracts, retinopathy and neuropathy. As used in the claims and specification hereof, treatment is meant to include both the prevention and alleviation of such conditions. The compound may be administered to a subject in need of treatment by a variety of conventional routes of administration, including orally, parenterally and topically. In general, these compounds will be administered orally or parenterally at dosages between about 0.5 and 25 mg./kg. body weight of the subject to be treated per day, preferably from about 1.0 to 10 mg./kg. However, some variation in dosage will necessarily occur depending on the condition of the subject being treated. The person responsible for administration will, in any event, determine the appropriate dose for the individual subject.

The novel compound of the invention may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups and injectable solutions. These pharmaceutical compositions can, if desired, contain additional ingredients such as flavorings, binders and excipients. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

Compounds of formula I may not only be advantageously employed for the preparation of aqueous pharmaceutical compositions for parenteral administration, as described above, but more particularly for the preparation of pharmaceutical compositions suitable for use as ophthalmic solutions. Such ophthalmic solutions are of principal interest for the treatment of diabetic cataracts by topical administration and the treatment of such conditions in this manner is a preferred embodiment of the present invention. Thus, for the treatment of diabetic cataracts the compounds of this invention are administered to the eye of the subject in need of treatment in the form of an ophthalmic preparation prepared in accordance with conventional pharmaceutical practice, see for example "Remington's Pharmaceutical Scienes" 15th Edition, pages 1488 to 1501 (Mack Publishing Co., Easton, Pa.). The ophthalmic preparation will contain a

compound of formula I or a pharmaceutically acceptable salt thereof in a concentration from about 0.01 to about 1% by weight, preferably from about 0.05 to about 0.5% in a pharmaceutically acceptable solution, suspension or ointment. Some variation in concentration will necessarily occur, depending on the particular compound employed and the condition of the subject to be treated, and the person responsible for treatment will determine the most suitable concentration for the individual subject. The ophthalmic preparation will preferably be in the form of a sterile aqueous solution containing, if desired, additional ingredients, for example preservatives, buffers, tonicity agents, antioxidants and stabilizers, nonionic wetting or clarifying agents and viscosity-increasing agents. Suitable preservatives include benzalkonium chloride, benzethonium chloride, chlorobutanol and thimerosal. Suitable buffers include boric acid, sodium and potassium bicarbonate, sodium and potassium borate, sodium and potassium carbonate, sodium acetate and sodium biphosphate, in amounts sufficient to maintain the pH at between about 6 and 8, preferably between about 7 and 7.5. Suitable tonicity agents are dextran 40, dextran 70, dextrose, glycerin, potassium chloride, propylene glycol and sodium chloride, such that the sodium chloride equivalent of the ophthalmic solution is in the range 0.9 plus or minus 0.2%. Suitable antioxidants and stabilizers include sodium bisulfite, sodium metabisulfite, sodium thiosulfite and thiourea. Suitable wetting and clarifying agents include Polysorbate 80, Polysorbate 20, Poloxamer 282 and Tyloxapol (Trade Mark). Suitable viscosity-increasing agents include dextran 40, dextran 70, gelatin, glycerin, hydroxyethylcellulose, hydroxymethylpropylcellulose, lanolin, methylcellulose, petrolatum, polyethylene glycol, polyvinyl alcohol, polyvinylpyrrolidone and carboxymethylcellulose. The ophthalmic preparation will be administered topically to the eye of the subject in need of treatment by conventional methods, for example in the form of drops or by bathing the eye in the ophthalmic solution.

The activity of the compounds of the present invention as agents for the control of chronic diabetic complications may be determined by a number of standard biological or pharmacological tests. Suitable tests include (1) measuring their ability to inhibit the enzyme activity of isolated aldose reductase; (2) measuring their ability to reduce or inhibit sorbitol accumulation in the sciatic nerve and lens of acutely streptozotocinized, i.e. diabetic, rats; (3) measuring their ability to reverse already-elevated sorbitol levels in the sciatic nerve and lens of chronic streptozotocin-induced diabetic rats; (4) measuring their ability to prevent or inhibit galacticol formation in the lens of acutely galactosemic rats; (5) measuring their ability to delay cataract formation and reduce the severity of lens opacities in chronic galactosemic rats; (6) measuring their ability to prevent sorbitol accumulation and cataract formation in isolated rat lens incubated with glucose; and (7) measuring their ability to reduce already elevated sorbitol levels in isolated rat lens incubated with glucose.

The present invention is illustrated by the following examples. It will be understood, however, that the invention is not limited to the specific details of these examples. Proton nuclear magnetic resonance spectra (NMR) were measured for solutions in perdeuterodimethyl sulfoxide (DMSO-$d_6$) and peak positions are expressed in parts per million (ppm) downfield from tetramethylsilane. The peak shapes are denoted as follows: s, singlet; d, doublet; t, triplet; q, quartet; m, multiplet; br, broad.

## Example 1

A. (+)7'-Fluoro-4'-hydroxy-3'-methyl-spiro[imidazolidine-4,1'-1',2',3',4'-tetrahydro naphthalene]2,5-dione

99.51 g (0.379 mol) of (±)7'-Fluoro-4'-hydroxy-3'-methyl-spiro[imidazolidine-4,1'-1',2',3',4'-tetrahydro-naphthalene]2,5-dione was combined with 171.0 g (0.398 mol) brucine dihydrate and was dissolved in 1.4 liter ethanol at reflux. The ethanol was filtered while hot to remove a small amount of insoluble material and was concentrated *in vacuo* to a foam. This foam was combined with 3.0 liters of acetonitrile at reflux. Some solid formed during this procedure. The reaction was allowed to cool to 23°C. The resultant solid was collected by filtration to give 128.96 g of solid: mp 222—224°C; $[\alpha]_D^{25} = -6.4°$ (methanol, C=1). In a similar manner from 20.34 g of the racemic alcohol was isolated 24.82 g of the brucine salt: mp 224—226°C; $[\alpha]_D^{25} = -5.8$ (methanol, C=1). The combined salts (153.78 g) were added to 1.5 litres of acetonitrile at reflux; 200 ml acetonitrile were boiled off and the reaction was allowed to cool to 23°C over 20 hours. The resulting solid was removed by filtration and dried to give 137.63 g: mp 226—227°C; $[\alpha]_D^{25} = 3.6°$ (methanol, C=1). This material was slurried in 1 liter boiling acetonitrile and allowed to cool to 23°C and the resulting solid was collected by filtration and dried to give 128.63 g: mp 227—228°C; $[\alpha]_D^{25} = +5.0°$ (methanol, C=1). The salt components were separated by slurrying in 1 liter of chloroform at 23°C for 1 hr. and then allowing the reaction to remain at 23°C for 20 hours. The resultant solid was collected by filtration and dried to give 45.45 g of the title compounds: mp 257.5—258.5°C; $[\alpha]_D^{25} = +120.1°$ (methanol, C=1).

The absolute configuration at the 4,1', 3' and 4' centers are assigned as 4,1'S, 3'R and 4'S based on a combination of single crystal x-ray, nuclear magnetic resonance and chemical interconversion studies.

B. 4,1'S Spir[imidazolidine-4,1'(2'H)-naphthalene]-2,5-dione 7'-Fluoro-3'-methyl (U = NH, n = 1, X = methyl, Y = F)

4,1'S 3'R 4'S Spiro[imidazolidine-4,1'-1',2',3',4'-tetrahydro-naphthalene]-2,5-dione-7'-fluoro-4'-hydroxy-3'-methyl in an amount of 26.4 g (0.1 mol) was combined with 30 mg p-toluenesulfonic acid in 124 ml toluene and heated at reflux for 20 hours. The reaction mixture was concentrated in vacuo and the residue was partitioned between the ethylacetate and water. The ethylacetate was washed with water and dried over anhydrous sodium sulfate. Removal of the ethyl acetate in vacuo gave 4,1'S spiro[imidazolidine-4,1'(2H)-naphthalene]-2,5-dione 7'-fluoro-3'-methyl- 21.09 g as a pale tan solid: mp 217—219°C. $[\alpha]_D^{25} = -7.3°$ (methanol, C=1). Anal. Calcd. for $C_{13}H_{11}N_2O_2F$: C, 63.41; H, 4.50; N, 11.38. Found: C, 63.11; H, 4.83; N, 11.14.

Example 2

A. (−) 7'-Fluoro-4'-hydroxy-3'-methyl-spiro[imidazolidine-4,1'-1',2',3',4'-tetrahydronaphthalene]2,5-dione

The acetonitrile mother liquors remaining after removal of 137.63 g of the solid having a m.p. of 226—227°C as described in Example 1A, $[\alpha]_D^{25} = +3.6°C$ ($CH_3OH$, C=1) were concentrated *in vacuo* to a foam. This foam was stirred with 1 liter of chloroform, a solid formed and was collected by filtration and washed thoroughly with five 1 liter portions chloroform and dried to give 48.22 g of the title compound: m.p. 253—254°C with decomposition; $[\alpha]_D^{25} = -114.2°$ ($CH_3OH$, C=1).

B. 4,1'R Spiro[imidazolidine-4,1'(2'H)-naphthalene]-2,5-dione 7'-fluoro-3'-methyl

4,1'R 3'S 4'R Spiro[imidazolidine-4,1'-1',2',3',4'-tetrahydronaphthalene]-2,5-dione-7'-fluoro-4'-hydroxy-3'-methyl in an amount of 13.2 g (49.9 mmol) was combined with 30 mg *p*-toluenesulfonic acid and 180 ml toluene and was heated at reflux for 4 hours. An additional 30 mg. *p*-toluenesulfonic acid was added and reflux was continued for an additional 27 hours. After cooling the toluene was decanted from a solid which was partitioned between 1N hydrochloric acid and ethylacetate. The ethylacetate was washed with 2 × 100 ml water and then dried over anhydrous sodium sulfate. After filtering to remove drying agent the solvent was removed in vacuo to give 4',R spiro[imidazolidine-4,1'(2'H)-naphthalene]-2,5-dione 7'-fluoro-3'-methyl as a golden solid −8.96 g: m.p. 214—216°C $[\alpha]_D^{25} = +3.0°$ (methanol, C=1). NMR ($Me_2SO$) 10.75 (very br s, 1), 8.45 (br s, 1), 6.67—7.33 (m, 3), 6.25 (s, 1), 2.38—2.70 (m, 2), 1.87 (br s, 3). Anal. Calcd. for $C_{13}H_{11}N_2O_2F$: C, 63.41; H, 4.50; N, 11.38. Found: C, 63.05; H, 4.78; N, 11.06.

Example 3

Spiro[imidazolidine-4,1'-[1H]indene]-2,5-dione

Spiro[imidazolidine-4,1'-[1H]indene-2,5-dione, 2',3'-dihydro in an amount of 2.02 g (10 mmol) was combined with 5.3 ml (20 mmol) bis(trimethylsilyl)trifluoroacetamide, and 1.6 g (10 mmol) bromine in 25 ml ethylenedichloride and heated at reflux to give a red solution. After 25 minutes at reflux the reaction color changed to a pale yellow. After an additional hour at reflux the reaction was cooled and concentrated in vacuo to give 2.81 g of an oil. This material was stirred at 100°C for 20 hours during which time a solid formed. Slurrying with ether gave a white solid which was collected by filtration and dried in vacuo to give 1.2 g spir[imidazolidine-4,1'-[1H]indene] 2,5-dione: mp 259—260°C with decomposition. This material was recrystallized from isopropyl alcohol to give 640 mg of the title compound as white crystals: m.p. 267—269°C with decomposition. NMR ($Me_2SO$) 11.0 (very br s, 1), 8.7 (br s, 1), 7.0—7.5 (m, 4), 6.93 (d, 1, J=5.5Hz), 6.30 (d, 1, J=5.5Hz).

Example 4

Spiro[imidazolidine-4,1'(2'H)-naphthalene]-2,5-dione

Spiro[imidazolidine-4,1'(2'H)-naphthalene]-2,5-dione, 3',4'-dihydro in an amount of 4.33 g (20 mmol) was combined with 10.4 ml bis(trimethylsilyl)trifluoroacetamide and 3.59 g (22.5 mmol) bromine in 40 ml chloroform and was heated at reflux for 20 min and then concentrated to an oil which was heated at 100°C until it solidified. This material was triturated with ether and the resultant solid was collected by filtration and dried to give 3.55 g spiro[imidazolidine-4,1'(2'H)-naphthalene]-2,5-dione: m.p. 195—205°C. NMR ($Me_2SO$) 13.0 (br s, 1), 8.47 (br, s, 1), 6.97—7.23 (m, 4), 6.53 (d, 1, J=10Hz), 5.77—6.2 (m, 1), 2.57—2.87 (m, 2).

Compounds of formula I of Examples 1—4 were tested for their ability to reduce or inhibit aldose reductase enzyme activity, following the procedure described in U.S. Patent 3,821,383 and based on the procedure of Hayman et al., Journal of Biological Chemistry, *240,* 877 (1965). The substrate employed was partially purified aldose reductase enzyme obtained from human placenta. The compounds were tested at levels at $10^{-4}$ to $10^{-6}$ M and were found to inhibit enzyme activity relative to untreated controls.

**Claims**

1. A compound of the formula

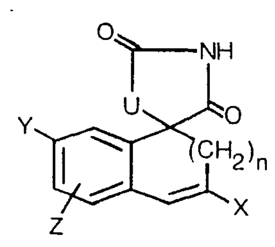

I

or a pharmaceutically acceptable salt thereof, wherein:

U is oxygen, sulfur or nitrogen substituted with hydrogen;

X is hydrogen or alkyl having 1 to 4 carbon atoms;

n is 0 or 1;

Y is hydrogen, halo, nitro, trifluoromethyl, hydroxy, alkoxy or alkylthio having 1 to 4 carbon atoms or alkyl having 1 to 4 carbon atoms; and

Z is hydrogen, halo, nitro, trifluoromethyl, hydroxy, alkoxy having 1 to 4 carbon atoms or alkyl having 1 to 4 carbon atoms, with the proviso that if either Y or Z is nitro the other is hydrogen.

2. A compound according to claim 1, characterized in that U is nitrogen substituted with hydrogen.

3. A compound according to claim 2, characterized in that X, Y and Z are each hydrogen.

4. A compound according to claim 2, characterized in that n is 1, X is alkyl having 1 to 4 carbon atoms, Y is fluoro and Z is hydrogen.

5. A compound according to claim 4, characterized in that X is methyl.

6. A compound according to claim 1, characterized in that U is oxygen, X is methyl, Y is fluoro and Z is hydrogen.

7. A compound according to claim 1, characterized in that U is sulfur, X is methyl, Y is fluoro and Z is hydrogen.

8. A pharmaceutical composition characterized by comprising a compound of claim 1 in an amount effective in the treatment of diabetic complications, and a pharmaceutically acceptable carrier.

**Patentansprüche**

1. Verbindung der Formel

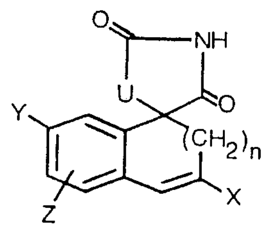

I

oder ein pharmazeutisch annehmbares Salz davon, worin:

U Sauerstoff, Schwefel oder mit Wasserstoff substituierter Stickstoff ist;

X Wasserstoff oder Alkyl mit 1—4 Kohlenstoffatomen ist;

n 0 oder 1 ist;

Y Wasserstoff, Halogen, Nitro, Trifluormethyl, Hydroxy, Alkoxy oder Alkylthio mit 1 bis 4 Kohlenstoffatomen oder Alkyl mit 1 bis 4 Kohlenstoffatomen ist; und

Z Wasserstoff, Halogen, Nitro, Trifluormethyl, Hydroxy, Alkoxy mit 1—4 Kohlenstoffatomen oder Alkyl mit 1 bis 4 Kohlenstoffatomen ist, mit dem Vorbehalt, daß wenn einer der Y oder Z Nitro ist, der andere Wasserstoff ist.

2. Verbindung nach Anspruch 1, dadurch gekennzeichnet, daß U mit Wasserstoff substituierter Stickstoff ist.

3. Verbindung nach Anspruch 2, dadurch gekennzeichnet, daß X, Y und Z jeweils Wasserstoff sind.

4. Verbindung nach Anspruch 2, dadurch gekennzeichnet, daß n 1 ist, X ein Alkyle mit 1—4 Kohlenstoffatomen ist, Y Fluor ist und Z Wasserstoff ist.

5. Verbindung nach Anspruch 4, dadurch gekennzeichnet, daß X Methyl ist.

6. Verbindung nach Anspruch 1, dadurch gekennzeichnet, daß U Sauerstoff ist, X Methyl ist, Y Fluor ist und Z Wasserstoff ist.

7. Verbindung nach Anspruch 1, dadurch gekennzeichnet, daß U Schwefel ist, X Methyl ist, Y Fluor ist und Z Wasserstoff ist.

8. Pharmazeutische Zusammensetzung, dadurch gekennzeichnet, daß sie eine Verbindung des Anspruchs 1, in einer bei der Behandlung diabetischer Komplikationen wirksamen Menge, und einen pharmazeutisch annehmbaren Träger umfaßt.

**Revendications**

1. Composé de formule:

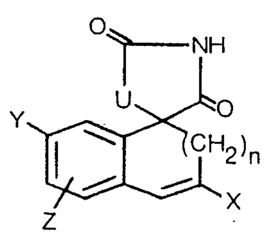

I

ou sel pharmaceutiquement acceptable d'un tel composé, dans laquelle:

U représente l'oxygène, le soufre ou l'azote substitué par de l'hydrogène;

X représente l'hydrogène ou un groupe alkyle ayant de 1 à 4 atomes de carbone;

n représente 0 ou 1;

Y représente l'hydrogène, un groupe halogéno, nitro, trifluorométhyle, hydroxy, alcoxy ou alkylthio ayant de 1 à 4 atomes de carbone ou un groupe alkyle ayant de 1 à 4 atomes de carbone; et

Z représente l'hydrogène, un groupe halogéno, nitro, trifluorométhyle, hydroxy, alcoxy ayant de 1 à 4 atomes de carbone ou un groupe alkyle ayant de 1 à 4 atomes de carbone, à condition que si l'un ou l'autre des groupes Y et Z est un groupe nitro, l'autre représente l'hydrogène.

2. Composé selon la revendication 1, caractérisé en ce que U représente l'azote substitué par de l'hydrogène.

3. Composé selon la revendication 2, caractérisé en ce que X, Y et Z représentent chacun l'hydrogène.

4. Composé selon la revendication 2, caractérisé en ce que n représente 1, X est un groupe alkyle ayant de 1 à 4 atomes de carbone, Y est un groupe fluoro et Z représente l'hydrogène.

5. Composé selon la revendication 4, caractérisé en ce que X est un groupe méthyle.

6. Composé selon la revendication 1, caractérisé en ce que U représente l'oxygène, X est un groupe méthyle, Y est un groupe fluoro et Z représente l'hydrogène.

7. Composé selon la revendication 1, caractérisé en ce que U représente le soufre, X est un groupe méthyle, Y est un groupe fluoro et Z représente l'hydrogène.

8. Composition pharmaceutique, caractérisée en ce qu'elle comprend un composé selon la revendication 1 en une quantité efficace pour le traitement des complications diabétiques, et un véhicule pharmaceutiquement acceptable.